# EUROPÄISCHE PATENTSCHRIFT

(11) **EP 0 969 783 B1**
(45) Veröffentlichungstag und Bekanntmachung des Hinweises auf die Patenterteilung: **29.05.2002**
(21) Anmeldenummer: 98952648.8
(22) Anmeldetag: 25.09.1998
(51) Int. Cl.: A61F 5/04

(54) **NOTFALL-STÜTZE ZUR STABILISIERUNG EINZELNER GLIEDMASSEN ODER KÖRPERPARTIEN**
EMERGENCY SUPPORT FOR STABILISING INDIVIDUAL LIMBS OR BODY PARTS
ELEMENT DE SOUTIEN DE SECOURS POUR STABILISER DES PARTIES DE CORPS INDIVIDUELLES

(30) Priorität: 28.09.1997 DE 19742833; 20.02.1998 DE 19807112
(43) Veröffentlichungstag der Anmeldung: 12.01.2000
(73) Patentinhaber: Stelzenmüller, Wolfgang, 63263 Neu-Isenburg (DE)
(72) Erfinder: Stelzenmüller, Wolfgang, 63263 Neu-Isenburg (DE)
(74) Vertreter: Luderschmidt, Schüler & Partner GbR
(86) Internationale Anmeldenummer: EP9806110
(87) Internationale Veröffentlichungsnummer: WO9916392

(56) Entgegenhaltungen:
- GB-A- 2 171 603
- US-A- 3 397 688
- US-A- 3 745 998
- US-A- 3 762 404

## Beschreibung

Die Erfindung geht aus von einer Notfall-Stütze gemäß dem Oberbegriff des Patentanspruches 1.

Die Lagesicherung eines Unfall-Verletzten bei der Bergung an einem Unfallort, etwa aus einem Kraftfahrzeug oder in unwegsamem Gelände, sowie für seinen Transport zur Klinik ist nach wie vor ein nur unbefriedigend gelöstes Problem. Insbesondere in den Fällen, in denen Schädel-, Nacken oder Rückenwirbelverletzungen angenommen werden müssen, ist die Vermeidung von Lageveränderungen bis zur entgültigen Klarheit über die Art der Behandlung in der Regel von lebenserhaltender Bedeutung. Zwar sind verschiedene Ausführungsformen sog. Bergeschienen bekannt und im Einsatz, die allerdings zum Teil sehr klobig und damit unhandlich und schwer anlegbar sind, in allen Fällen jedoch für das Anlegen die vorherige, in der Regel mit erheblichen Lageveränderungen des Verletzten verbundene Bergung des Verletzten erfordern.

Entsprechendes gilt für eine Reihe von operativen Eingriffen im Kopf- und Halsbereich, beispielsweise das Anlegen eines Luftröhrenschnittes, während denen eine absolut stabile Lage über längere Zeit hinweg absolut sichere Lage Stabilisierung des Halsbereiches und Kopfes gewährleistet werden muß. Die für diesen Zweck in den Operations-Sälen eingesetzten Gurtsysteme vermögen aufgrund ihrer Flexibilität diese Forderung nur ungenügend zu erfüllen.

Es ist beispielsweise aus dem DE-GM G 83 12 991 ein Stützverband für die Halswirbelsäule bekannt geworden, der aus mehreren miteinender verbundenen und gegeneinander abgegrenzten schlauchartigen Manschetten bestehen, die mit einem Granulat gefüllt sind und nach dem Anlegen in einer den Hals- und Kopfbereich im wesentlichen umschließenden Weise evakuiert werden könnne, so daß der Hals- und Kopfbereich fest in die Manschetten eingebettet sind. Dieser bekannte Stützverband hat eine Reihe wesentlicher Nachteile. Ein Nachteil ist insbesondere in der gegenseitigen Abgrenzung der einzelnen Manschetten zu sehen, die einerseits unter entsprechender Verzögerung des Anlegens des Stützverbandes die Evakuierung in einem Arbeitsgang und andererseits den Übertritt von Granulat aus einer Manschette in die benachbarte und damit ihre Anpassung an die jeweils situationsbedingt geforderte Form nicht nur wesentlich behindert, sondern praktisch ausschließt. Hinzu kommt, daß die Notfallstütze aufgrund der Verbindung der einzelnen Manschetten auf ihrer gesamten Länge die gestützte Körperpartie, insbesondere jedoch den Halsbereich des Patienten vollständig umschließt und damit den Zugang für gegebenenfalls lebensnotwendige Eingriffe, etwa einen Luftröhrenschnitt ausschließt. Die bekannte Manschette erscheint damit für den Einsatz in Notfallsituationen, die einerseits eine außerordentliche Flexibilität, eine schnelle und problemlose Handhabung und den Zugang zum Halsbereich ohne wesentlichen Verlust an Stützwirkung erfordert, ungeeignet und hat daher auch Eingang weder in die Notfall- noch in die stationär-medizinische Praxis gefunden.

Die US-3,745,998 beschreibt eine unterstützende Struktur aus einem evakuierbaren, mit Granulat gefüllten Kissen, das mittels zweier Säume in zwei Regionen unterteilt ist, so daß innerhalb des Kissens zwei einzelne Innenräume ausgebildet sind. Die beiden Innenräume sind über eine Passage zwischen den beiden Säumen miteinander verbunden. Die Passage ist derart ausgelegt, daß der Durchgang von Luft möglich ist, der Durchgang des Granulates allerdings verhindert wird.

Die US-3,762,404 beschreibt eine evakuierbare, mit Granulat gefüllte, kissenförmige Positionierungshilfe, wobei das Kissen durch Trennwände in einzelne Kammern unterteilt ist. In den Trennwänden sind Luftlöcher vorgesehen, die den Durchfluß von Luft erlauben, wohingegen ein Durchgang des Granulates nicht möglich ist.

Sowohl die unterstützende Struktur als auch die kissenförmige Positionierungshilfe haben den Nachteil, daß diese nur bedingt in die situationsbedingt erforderliche Form gebracht werden können, so daß das Anlegen der beschriebenen Vorrichtungen erschwert ist.

Der vorliegenden Erfindung liegt demgemäß als Aufgabe die Schaffung einer Notfall-Stütze zugrunde, die ein einfaches Anlegen auch unter schwierigen Umfeld-Verhältnissen erlaubt und eine absolut sichere Lagestabilisierung des gestützten Körperbereiches bei erforderlichenfalls gutem Zugang zu den sensiblen Körperbereichen, insbesondere dem Halsbereich, erlaubt.

Diese Aufgabe wird mit einer Notfall-Stütze mit den im Patentanspruch 1 wiedergegebenen Merkmalen gelöst.

Durch die Erfindung ist eine Notfall-Stütze geschaffen, die in ihrer Gesamtheit in einem Arbeitsgang evakuierbar und daher schnell und problemlos in ihre Hartschalenform gebracht werden kann, wobei einerseits zwar der Fluß des Granulates aus einem Bereich in andere Bereiche behindert, d.h. eine hohe Formstabilität auch unter erschwerten Arbeitsbedingungen gewährleistet, jedoch ohne wesentliche Probleme möglich ist, so daß insbesondere zum Anlegen eine Anpassung an die die sich aus der jeweiligen örtlichen und persönlichen Situation ergebenden Notwendigkeiten ohne weiteres möglich ist. Die erfindungsgemäße Notfallstütze kann in jede beliebige Form und damit unter Nutzung kleinster Freiräume bzw. Durchlässe in Stellung gebracht und der Körperform angepaßt und danach mit dieser der Körperform angepaßten Kontur mit einem einzigen Evakuierungsvorgang zu einer starren und geschlossenen Stütze verfestigt werden. Das Anlegen der Stütze kann somit auch unter schwierigen Umfeldbedingungen oder in unwegsamem Gelände vorgenommen werden, so daß Lageveränderungen vom Beginn der Bergung des Verletzten an bis zu seiner stationären Lagerung weitestgehend ausgeschlossen werden können.

Weitere Einzelheiten der Erfindung werden nachstehend anhand der beigefügten Zeichnung erläutert. Es zeigen
- Fig. 1: die Ansicht einer cervivalen, d.h. der Stabilisierung des Kopf- und Nackenbereiches dienenden Notfall-Stütze
- Fig. 2: eine Sicht von der Seite auf Fig. 1
- Fig. 3: die Rückansicht der in Fig. 1 wiedergegebenen Notfallstütze
- Fig. 4: die Ansicht einer Kinnstütze
- Fig. 5: eine Sicht von der Seite auf Fig. 4
- Fig. 6: die Seitenansicht eines Kopfes mit Notfallstütze
- Fig. 7: die schematische Darstellung eines Befestigungsgurtes für das Filter-System eines Beatmungsgerätes
- Fig. 8: die Ansicht eines Kopfes mit angelegter Notfall-Stütze mit Filter-Systems für ein Beatmungsgerät
- Fig. 9: die Ansicht einer thoracalen, d.h. der Stabilisierung des Brust- und Lendenwirbelbereiches dienenden Notfall-Stütze
- Fig. 10: die Rück-Ansicht einer Trage bzw. Bergeplatte zur Bildung eines Rettungsgerätes
- Fig. 11: die schematische perspektivische Wiedergabe eines Bezuges für eine Bergeplatte mit thoracaler Notfall-Stütze
- Fig. 12: eine vollständige Wiedergabe eines Rettungsgerätes
- Fig. 13: die schematische Schnittdarstellung einer Ausführungsform eines Fahrzeugsitzes mit integrierter Bergeplatte
- Fig. 14: die schematische Schnittdarstellung eines gleichzeitig die feste Struktur eines Bergesystems bidenden Fahrzeugsitzes
- Fig. 15: die perspektivische Darstellung eines Hebebockes zur Hebung des Bergesystems aus einem Fahrzeug
- Fig. 16: die schematische Darstellung des Hebevorganges

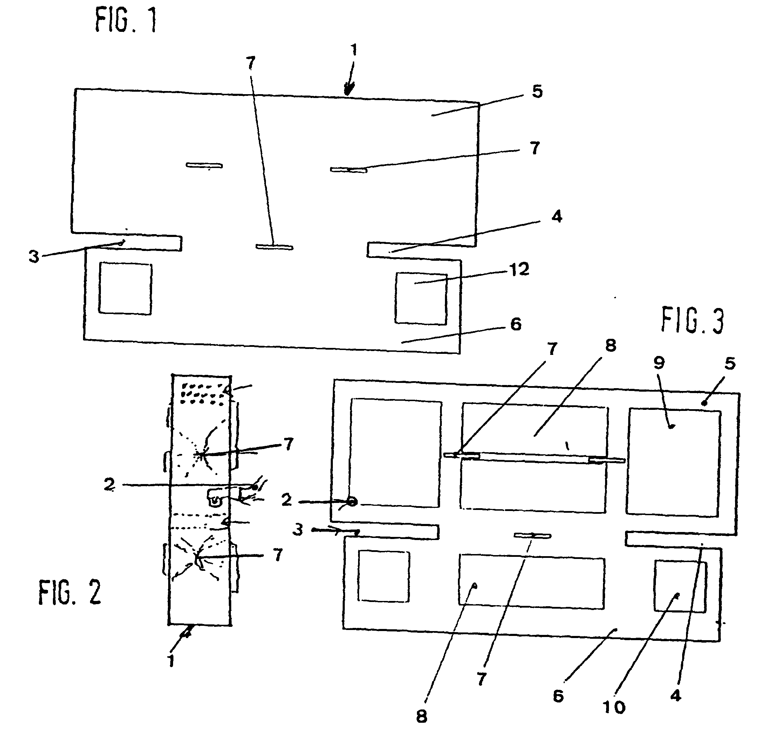

In den 1 bis 6 ist eine cervicale, d.h. der Stabilisierung des Kopf- und Nackenbereiches eines Menschen dienende Notfallstütze wiedergegeben, die im wesentlichen aus einem mit einem Granulat gefüllten sowie zum Zwecke des abwechselnden Evakuierens und Flutens mit einem Ventil 2 versehenen Kissen 1 aus zwei an ihren Rändern verbundenen Bahnen aus luftdichtem flexiblem Material. Im Falle des ein Cervical-Kissen wiedergebenden Ausführungsform ist das Kissen mittels in einer Flucht liegender seitlicher Einschnitte 3, 4, in zwei Segmente 5, 6 unterteilt ist, von denen das Segment 5 der Umfassung der Schädels und das andere Segment 6 der Umfassung des Nacken-Hals-Bereiches dienen, wobei in dem zwischen den Einschnitten 3, 4 gelegenen Bereich eine sowie in dem größeren der beiden Segmente 5 zwei weitere - in Flucht zueinander liegende - Querschnittsverengungen in Form strichförmiger Verschweißungen 7 als Rieselsperren vorgesehen sind, durch die das Kissen in mehrere - im Beispielsfalle drei - Zonen unterteilt wird, zwischen denen der Fluß des Granulates behindert ist. Auf diese Weise kann zwar durch entsprechenden äußeren Druck das Granulat von einer Zone in die andere gedrückt und dem Kissen die für den Einzelfall, etwa beim Anlegen in einem beengten Bewegungsraum, benötigte Form gegeben werden, die es dann während des weiteren Arbeitens aufgrund der durch die Rieselsperren bewirkten Behinderung des freien Flusses zumindest im wesentlichen während der kritischen Anlegephase beibehält. Aus der in schematischer Weise die Verschweißung 7 wiedergebenden Fig. 2 ist deutlich erkennbar, daß durch die Verbindung aufgrund des starken Einzuges um die Schweißnaht herum eine weit über deren Länge hinausgehende Verengung des Fließquerschnittes erzielt wird.

Es ist weiterhin das Kissen 1 - siehe insbesondere Fig. 3 - im Mittelbereich seiner nach dem Anlegen dem Körper abgewendeten Außenfläche mit einem im Beispielsfalle aus drei Flecken 8 bestehenden Besatz aus Klettband als Teil eines Klett-Flausch-Verbinders versehen, der der direkten Fixierung des Kissens 1 an einer mit Flauschbesatz versehenen festen Struktur oder zur Anbringung einer Flauschbesatz tragenden Magnet-, Haft-PVC- oder dergl. Haft-Folie dienen kann, mit deren Hilfe es auf einer eine entsprechende Oberfläche aufweisenden festen Struktur, etwa einem Operations-tisch oder - zahnärztlichen - Behandlungsstuhl fixierbar ist. Es hat sich gezeigt, daß durch das Anlegen alleine einer evakuierbaren Granulat-Manschette eine ausreichende Immobilisierung des Körpers bzw. einzelner Körperpartien nicht erreicht werden kann, vielmehr eine in kritischen Phasen unerwünschte und gegebenenfalls gefährliche Verwindung möglich ist, die durch die Stabilisierung des Kissens mittels einer festen Struktur zuverlässig verhindert werden kann, so daß erst auf diese Weise das angestrebte Ziel der ausreichenden Immobilisierung des Körpers erreicht wird. Hierbei wird durch die Anbringbarkeit von Haftfolien unterschiedlicher Wirkungsweise eine große Flexibilität erreicht derart, daß jede vorhandene feste Struktur, d.h. etwa im Notfall eine gerade verfügbare Metall- oder Kunststoff-Platte oder im stationären oder ambulanten Betrieb der vorhandene Operationstisch bzw. Behandlungsstuhl herangezogen werden kann. Es werden auf diese Weise die Verwendungsmöglichkeiten der Notfall-Stütze wesentlich erweitert.

Weitere Haftflecken, etwa Klett-Flecks 9, 10 auf der Außenseite des Kissens 1 dienen der Anbringung von (Flausch)-Spannbändern 11 über die Stirn-, Augen- und Kinnpartie und damit der weiteren Stabilisierung des Kopfes in dem ihn umfassenden Kissen.

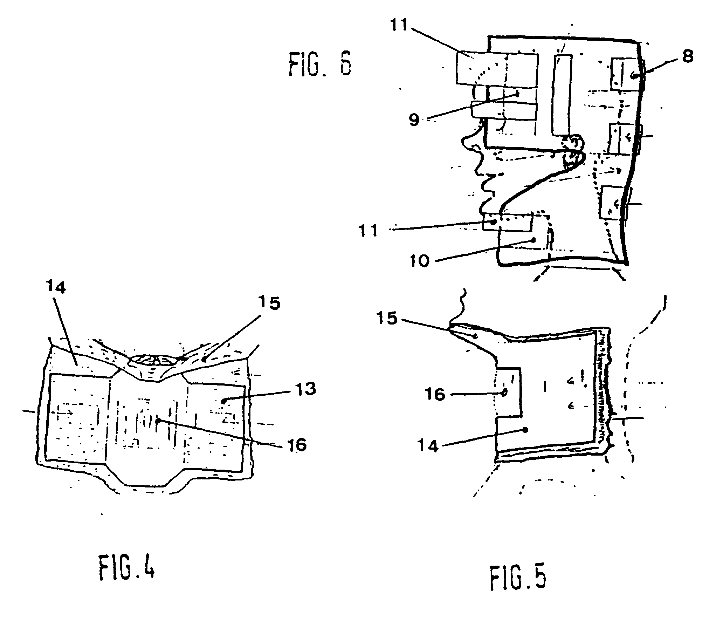

Das Kissen ist weiterhin - siehe Fig. 1 - auf seiner inneren Seite mit Flauschflecken 12 eines Klett-Flausch-Verbinders versehen zur Befestigung der in den Fig. 4 und 5 wiedergegebenen, den entsprechenden Klett-Fleck 13 des Verbinders tragenden Kinnstütze 14 versehen. Sie wird auf diese Weise fest in die Gesamtanordnung eingebunden, wobei durch die konkave Wölbung der Kinnplatte 15 eine Drehstabilisierung des Kopfes und in Verbindung mit der Fixierung des Kissens an einer festen Struktur dessen vollständige Immobilisierung erreicht wird. Darüberhinaus kann die sich gegen den Brustbereich des Körpers abstützende Kinnstütze selbst als eine feste Struktur angesehen werden, die in Verbindung mit dem über die Kinnplatte 15 wirksamen Schädel für die Immobilisierung des kritischen Halswirbelbereiches Sorge trägt oder zumindest wesentlich zu seiner Immobilisierung beiträgt. Die Kinnstütze ist in ihrem unterhalb der Kinnplatte 15 gelegenem Bereich mit einem Fenster 16 versehen, durch das hindurch evtl. notwendige Diagnose- oder Hilfsmaßnahmen im Halsbereich, also etwa das Ertasten des Karottis-Pulses, zum Legen eines Luftröhren-Schnittes oder dergl., vorgenommen werden können.

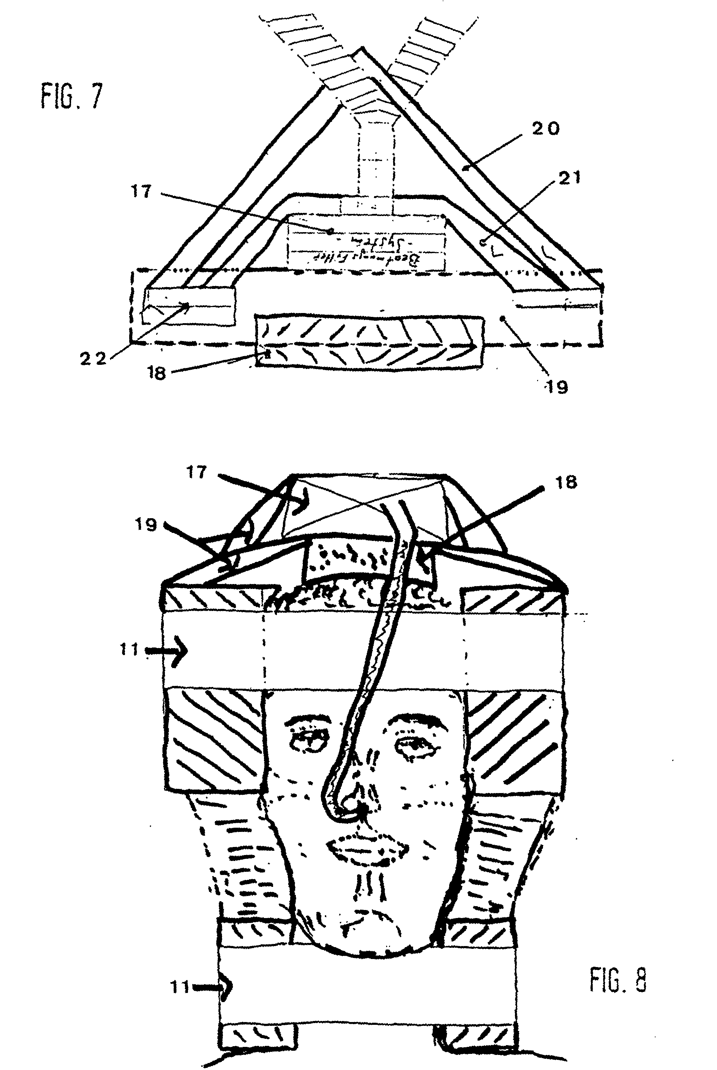

Der (Klett)-Haftfleck 9 bzw. ein entsprechend gesonderter Haftfleck dient weiterhin - siehe die Fig. 7 und 8 - zur Fixierung des Filtersystems 17 eines Beatmungsgerätes, zu welchem Zweck ein mit einem Kopfpolster 18 versehenes Flauschband 19 vorgesehen ist, an dessen dem Kopfpolster abgewendeten Fläche einseitig im Beispielsfalle zwei Flauschbänder 20, 21 fest vernäht sind, zu deren Fixierung auf der Gegenseite ein Klett-Fleck 22 angebracht ist. Zur Befestigung des Filters wird zunächst das das Kopfpolster 118 tragende Flauschband 19 über den Kopf gespannt und an den Flecken 9 des Kissens 1 fixiert und danach die Teile des Filtergerätes mittels der Bänder 20, 21 durch Fixierung gegen den Klett-Fleck 22 befestigt.

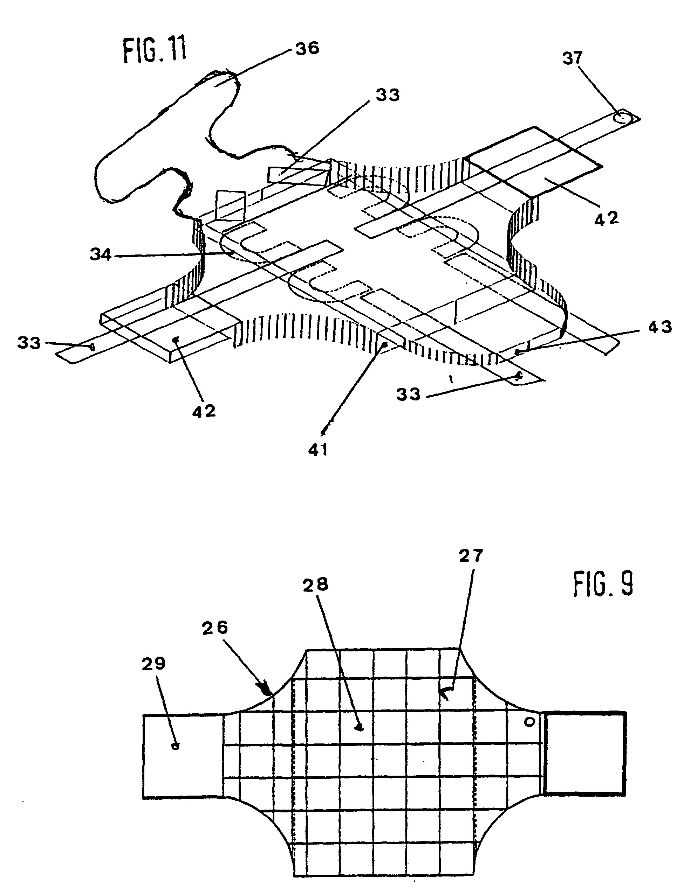

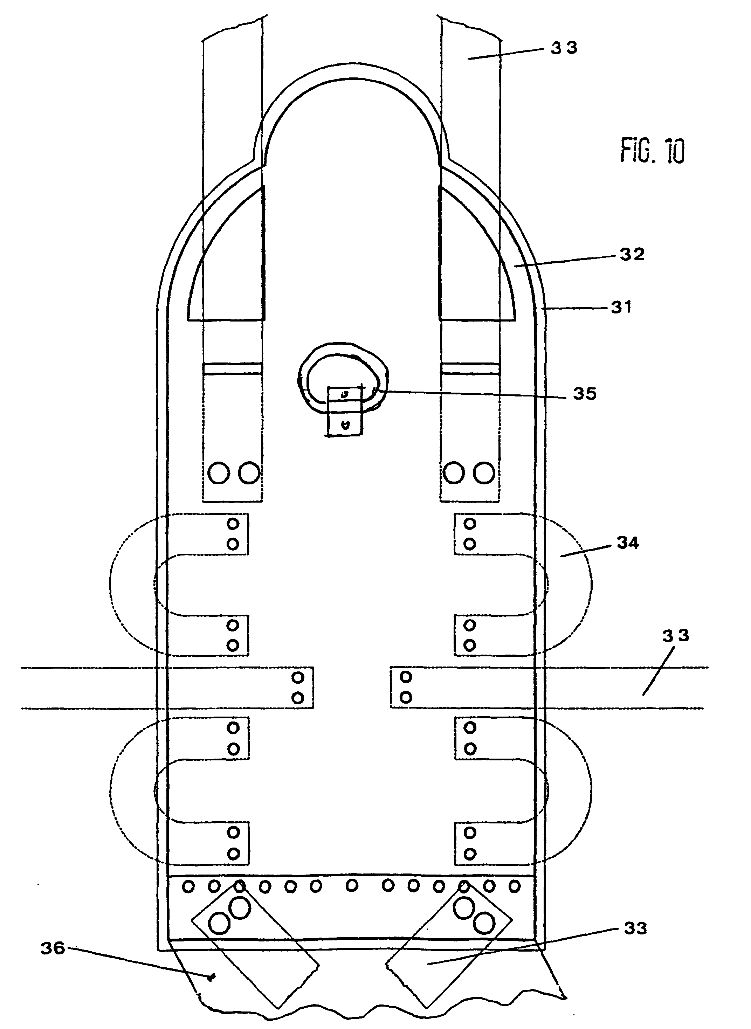

Die in Fig. 9 wiedergegebene Notfall-Stütze dient der Stabilisierung des Brust- und Lendenwirbel-Bereiches und besteht aus einem Kissen 26 mit im wesentlichen kreuzförmiger Kontur, das durch Steppoder Schweißnähte 27 nach Art einer Steppdecke in Felder 28 unterteilt und mit seitlichen, auf ihren beiden Flächen wechselseitig Klett- und Flausch-Flecken eines Klett-Flausch-Verbinders tragenden Laschen 29 versehen ist. Es weist im übrigen die - nicht dargestellt - einen den Flecken 8 entsprechenden Besatz zur Fixierung an einer festen Struktur auf, die - siehe Fig. 10 - beispielsweise von einer mit Flauschbesatz 31 beschichteten Bergeplatte 32 oder Trage aus Kunststoff, vorzugsweise Kevlar-Aramid, gebildet sein kann, an der die Stränge eines Sechspunktgurtes 33 mit einer die Umschließung der Kissens einschließlich des gestützten Patienten erlaubenden Länge, weiterhin Tragegurte 34 sowie eine Öse 35 zum Einklinken eines Kranhakens angeordnet sind. An der Bergeplatte 32 ist weiterhin ein im wesentlichen T-förmiges bzw. Doppel-T-förmiges Beckentuch 36, das zum Bergen und Transport von unten durch den Schritt des Patienten nach vorn gezogen und dort zusammen mit den Gurten 33 fixiert werden kann.

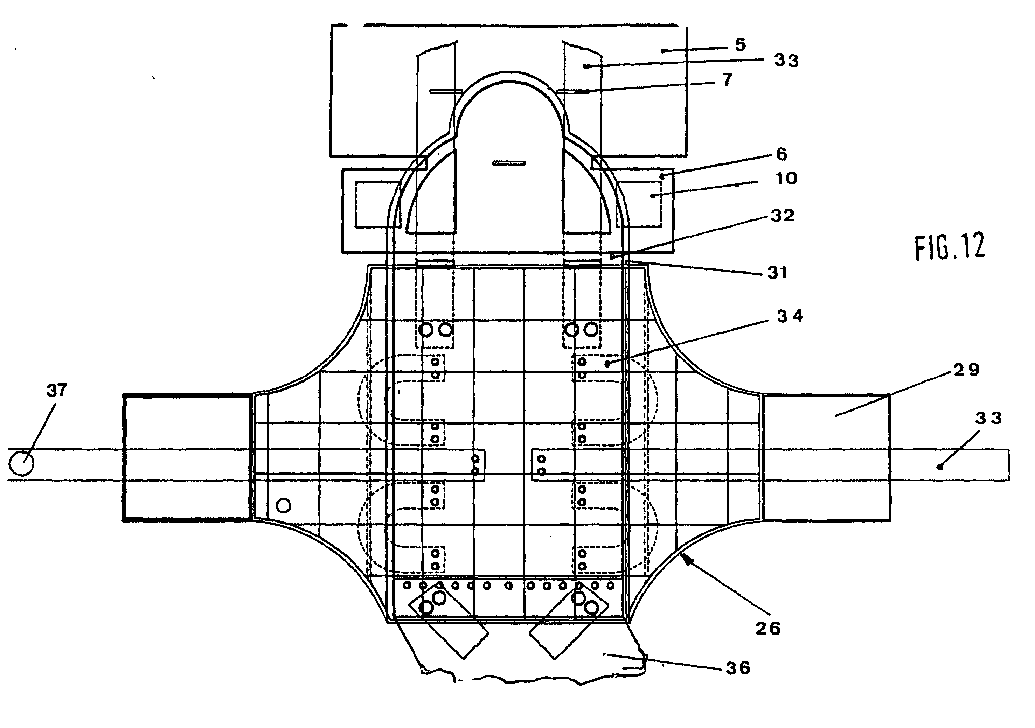

Eine Abwandlung der in den Fig 9 und 10 wiedergegebenen Ausführungsform eines Bergegerätes ist in Fig. 11 wiedergegeben. In diesem Falle sind die im wesentlichen kreuzförmige Thoracal-Stütze sowie die Bergeplatte mit miteinander verbindbaren bzw. verbundenen, den Flauschbzw. Klett-Besatz tragenden Bezügen 41, 43 versehen, wobei der die kreuzförmige Thoracal-Stütze umgebende Bezug 41 die seitlichen, auf ihren beiden Flächen wechselseitig Klett- und Flausch-Flecken eines Klett-Flausch-Verbinders tragenden Laschen 42 trägt, während der die Bergeplatte umgebende Bezug 43 mit Öffnungen für den Durchtritt der Gurte 33, der Trageschlaufen 34, der Kran-Öse 35 sowie des Beckentuches 36 aufweist. Die Gurte enden vorteilhaft in einem Zentralschloß 37.

Fig. 12 zeigt das Bergegerät in seiner Gesamtheit, das eine absolute Immobilisierung eines Verletzten und dessen weitgehend von Risiken befreite Bergung selbst unter Einsatz von maschinellem Bergegerät, etwa einem zur Verfügung stehenden Werkstatt- oder Fahrzeug-Kran erlaubt, dessen Haken in die Kran-Öse 35 eingehängt wird, worauf der auf der Bergeplatte mittels der auf ihr fixierten cervicalen und der thoracalen Notfall-Stützen 1, 26 mit Kinnstütze 14 und Spannlaschen 29 bzw. 42 in Verbindung mit dem Beckentuch 36 und dem Sechspunkt-Gurt stabilisierte Patient auch aus schwieriger Lage maschinell gehoben werden kann, ohne daß eine Verwindung oder insbesondere Versetzung im Wirbelsäulenbereich befürchtet werden muß.

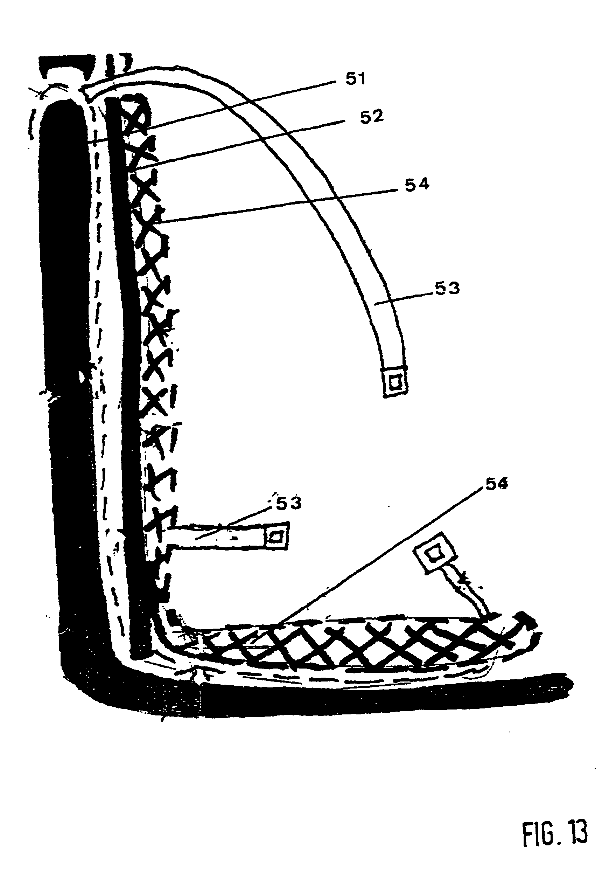

Im Falle der in Fig. 13 wiedergegebenen Ausführungsform ist die feste Struktur von einer in einen Fahrzeugsitz 51 integrierten Platte 52 gebildet, wobei die beispielsweise aus Kevlar-Aramid oder Glasfaser-Material hergestellte Platte 52 einerseits eine der Sitz-Ergonomie entsprechende Form aufweist, weiterhin mit einer sitzüblichen Polsterung 54 sowie mit Bergegurten 53 versehen ist, die in der üblichen Sitz-Gebrauchsstellung an einer hierfür geeigneten, d.h. den Sitzkomfort nicht beeinträchtigenden Stelle, beispielsweise in eigens hierfür in der Polsterung 54 der Bergeplatte 52 oder im Fahrzeugsitz 51 vorgesehenen Hohlräumen (verdeckt) untergebracht sind. Das Bergen einer Person aus einem Unfall-Fahrzeug gestaltet sich in diesem Fall ausgesprochen einfach und praktisch ohne Lageveränderung der (verletzten) Person in der Weise, daß zunächst die Person mittels der aus der Polsterung freigeschnittenen Bergegurte gegen die Platte 52 fixiert, danach der die Person am Fahrzeugasitz festhaltende fahrzeug-übliche Sicherheitsgurt an einer beliebigen zugänglichen Stelle gekappt und die die Bergeplatte 52 auf dem Sitz 51 haltende Verbindung gelöst und die Bergeplatte 51 zusammen mit der auf ihr fixierten Person aus dem Fahrzeug gehoben werden.

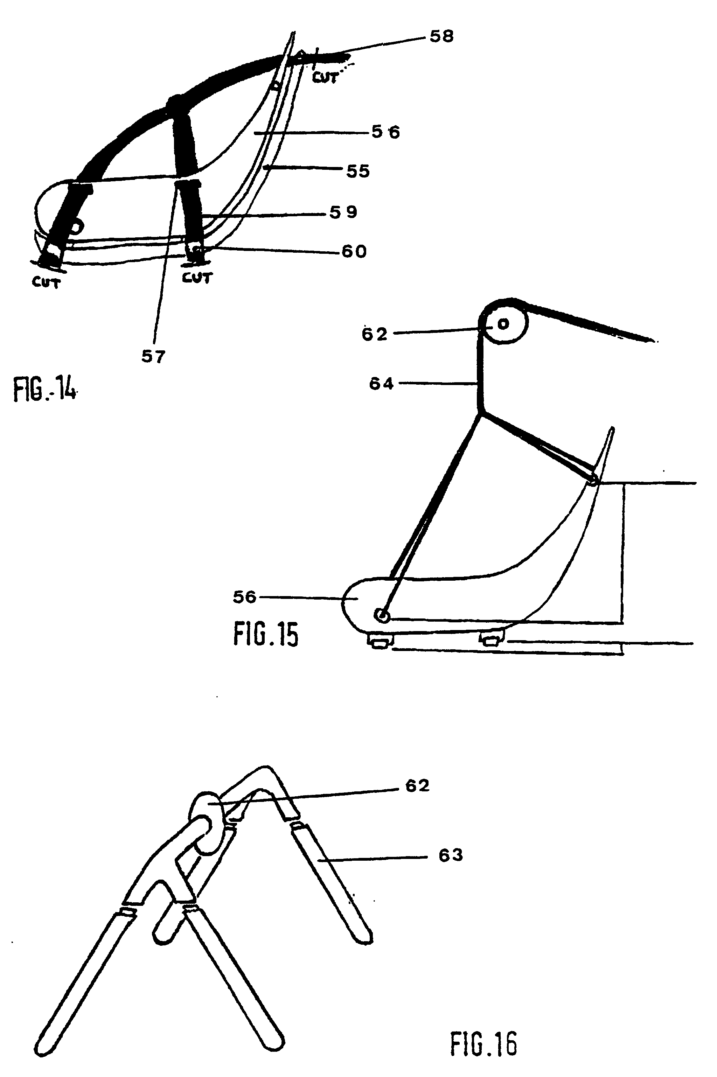

Anstelle einer sandwichartigen Verbindung einer Bergeplatte mit einem Fahrzeugsitz kann auch in der in Fig. 14 schematisch wiedergegebenen Weise die Sitzschale 55 selbst als feste Struktur des Bergesystems herangezogen und in der anhand von Fig. 13 beschriebenen Weise mit einem gesonderten Bergegurt ausgerüstet sein, an dessen Stelle jedoch auch die fahrzeugfest installierten Sicherheitsgurte nutzbar gemacht werden können derart, daß die Sitzschale 55 bzw. die mit ihr verbundene Platte 56 Schlitze 57, 58 zum Durchzug der Gurte 59 aufweisen, die auf der vom Benutzer abgewendeten Seite des Sitzes 55 bzw. der Platte 56 mit Beschlägen 60 versehen sind, mit deren Hilfe die Gurtenden am Fahrzeugsitz bzw. der Platte fixiert bzw. miteinenander verbunden werden können. In diesem Falle ist es in der Bergesituation lediglich erforderlich, die durch die Schlitze geführten Gurte zwischen Befestigungsbeschlägen und ihrer im Fahrzeug gelegenen Befestigungsstelle zu Schneiden, mit mit den Beschlägen 60 in entsprechende an Fahrzeugsitz angebrachten Aufnahmen - nicht sichtbar - einzuhängen und den Gurt mittels der - in der Regel im vorderen Gurtschloß - integrierten Spanneinrichtung nachzuspannen, um die verletzte Person aus ihrer Verklmmerung mit dem Fahrzeug zu lösen und unbeweglich auf der festen Struktur - Fahrzeugsitz bzw. Platte - zu fixieren. Zur weiteren Vereinfachung der Bergungsarbeiten ist zweckmäßig der Fahrzeugsitz in den Fällen, in denen er als Ganzes als feste Struktur des Bergesystems dient, mittels Schnellspann-Verschlüssen am Fahrzeugboden befestigt.

Die Bergung erfolgt danach - gegebenenfalls nach Öffnung des Daches - unter Zuhilfenahme eines - siehe die Fig. 15 und 16 - auf das Fahrzeug aufgesetzten oder dieses überspannenden, eine Rolle 62 aufweisenden Hebebockes 63 mittels eines in den Sitz eingehängten Seilzuges 64.

## Patentansprüche

1. Notfall-Stütze zur Stabilisierung einzelner Gliedmaßen oder Körperpartien für den Transport oder die Durchführung operativ-therapeutischer Maßnahmen, aufweisend ein mit einem Granulat gefülltes sowie zum Zwecke des abwechselnden Evakuierens und Flutens mit einem Ventil (2) versehenes, die zumindest teilweise Umhüllung der Fluidmaßen bzw. der Körperpartien erlaubendes Kissen (1, 26) aus zwei an ihren Rändern verbundenen Bahnen aus luftdichtem flexiblem Material, wobei das Kissen mit einem Besatz (8) aus Klettband als Teil eines Klett-Flausch-Verbinders versehen ist, wobei in dem das Granulat enthaltenden Kissseninnenraum Schikanen bzw. Drosseln (7) zur Behinderung des freien Granulatflusses und damit zur Erhaltung der einsatzbedingt vorgenommenen Granulatverteilung vorgesehen sind, **dadurch gekennzeichnet, daß** eine mit Flauschbesatz versehene feste Struktur vorgesehen ist, daß der Besatz (8) im Mittelbereich der nach dem Anlegen der zu stabilisierenden Fluidmaßen oder Körperpartien abgewandten Außenfläche des Kissens (1, 26) vorgesehen ist zur direkten Fixierung des Kissens an der festen Struktur, wobei die feste Struktur von eines Bergeplatte, Frage, Sitzschale eines Fahrzeugsitzes oder eine mit des Sitzschale eines Fahrzeugsitzes verbundenen bzw. in diesen integrierten Platte gebildet ist.

2. Notfall-Stütze nach Anspruch 1, **dadurch gekennzeichnet, daß** die Bahnen des Kissen zur Verengung der Fließquerschnitts in ihren Innenbereichen punkt- oder linienförmig miteinander verbunden sind.

3. Notfall-Stütze nach Anspruch 1 oder 2, **dadurch gekennzeichnet, daß** das Kissen aus thermoplastischem Material besteht und seine Bahnen durch gegenseitige Verschweißung punkt- oder linienförmig miteinander verbunden sind.

4. Notfall-Stütze nach einem der Ansprüche 1 bis 3, **dadurch gekennzeichnet, daß** das Kissen mittels in einer Flucht liegender seitlicher Einschnitte (3, 4) in zwei Segmente unterteilt ist, von denen das eine Segment der Umfassung des Schädels und das andere der Umfassung des Nacken-Hals-Bereiches dient und in dem zwischen den Einschnitten (3, 4) gelegenen Bereich mit mindestens einer den freien Granulatfluß behindernden Querschnittsverengung (7) versehen ist.

5. Notfall-Stütze nach Anspruch 4, **dadurch gekennzeichnet, daß** das Kissen auf der Innenseite des den Nacken-Hals-Bereich umfassenden Segmentes (6) sowie auf der Außenseite des den Schädel umfassenden Segmentes (5) mit Klett- oder Flauschflecken (9, 10, 12) eines Klett-Flausch-Verbinders versehen ist zur lösbaren Befestigung eines weiteren mit dem entsprechenden Gegenbesatz versehenen Hilfsgerätes, insbesondere einer Kinnstütze (14) und/oder des Filtersystem (17) eines Beatmungsgerätes.

6. Notfall-Stütze nach Anspruch 5, **dadurch gekennzeichnet, daß** zur Befestigung des Filtersystems (17) ein mit einem Kopfpolster (18) versehenes, an entsprechenden Klettflecken (9) des Kissens (1) fixierbares Flauschband (19) vorgesehen ist, an dessen dem Kopfpolster (18) abgewendeter Fläche einseitig mindestens ein Flauschband (20, 21) fest vernäht ist und auf dessen anderer Seite ein Klett-Fleck (22) zur Fixierung des Flauschbandes bzw. der Flauschbänder (20, 21) angebracht sind.

7. Notfall-Stütze nach Anspruch 5, **dadurch gekennzeichnet, daß** ferner eine Kinnstütze (14) mit einem Gegenbesatz vorgesehen ist, die lösbar an dem Kissen (1) befestigtbar ist, wobei die Kinnstütze (14) in ihrem unterhalb der Kinnplatte (15) gelegenem Bereich mit einem Fenster (16) zur Durchführung von Diagnose- oder Hilfsmaßnahmen versehen ist.

8. Notfall-Stütze nach einem der Ansprüche 1 bis 3, **dadurch gekennzeichnet, daß** das Kissen von einer nach Art einer Steppdecke in geschlossene Felder unterteilten Matte aus luftdurchlässigem Material gebildet ist, die eine das Verschlußventil enthaltende Umhüllung aufweist.

9. Notfall-Stütze nach einem der Ansprüche 1 bis 8, **dadurch gekennzeichnet, daß** das Kissen an den seitlichen Kanten ihrer Außenfläche mit mehreren Klett- oder Flauschband-Flecken eines Klett-Flausch-Verbinders versehen ist.

10. Notfall-Stütze nach Anspruch 8 oder 9, dadurch gekenzeichnet, daß das Kissen (26) eine im wesentlichen kreuzförmige Kontur besitzt und mit seitlichen, auf ihren beiden Flächen wechselseitig Klett- und Flausch-Flecken eines Klett-Flausch-Verbinders tragenden Laschen (29) versehen ist.

11. Notfall-Stütze nach eine der mehreren der Ansprüche 1 bis 10, **dadurch gekennzeichnet, daß** die feste Struktur von einer mit Flauschbesatz (31) beschichteten Bergeplatte oder Trage (32) aus Kunststoff, vorzugsweise Kevlar-Aramid, gebildet ist, an der die Gurte eines Sechspunktgurtes (33) mit einer die Umschließung des Kissens einschließlich des gestützten Patienten erlaubenden Länge, Trageschlaufen (34) sowie eine Kran-Öse (35) angeordnet sind, wobei das Kissen (1) an der festen Struktur fixierbar ist.

12. Notfall-Stütze nach Anspruch 11, **dadurch gekennzeichnet, daß** die im wesentlichen kreuzförmige Thoracal-Stütze (26) sowie die Bergeplatte mit miteinander verbindbaren bzw. verbundenen, den Flausch- bzw. Klett-Besatz tragenden Bezügen (41, 43) versehen sind, wobei der die kreuzförmige Thoracal-Stütze umgebende Bezug (41) die seitlichen, auf ihren beiden Flächen wechselseitig Klett- und Flausch-Flecken eines Klett-Flausch-Verbinders tragenden Laschen (42) trägt, während der die Bergepaltte umgebende Bezug (43) mit Öffnungen für den Durchtritt der Gurte (33), der Trageschlaufen (34), der Kran-Öse (35) sowie des Beckentuches (36) aufweist.

13. Notfall-Stütze nach Anspruch 12, **dadurch gekennzeichnet, daß** der Sechspunktgurt (33) mit einem Zentralschloß (37) versehen ist.

14. Notfall-Stütze nach Anspruch 11, **dadurch gekennzeichnet, daß** die Sitzschale (51, 55) des Fahrzeugsitzes bzw. die mit ihr verbundene Platte (52, 56) mit Schlitzen (57, 58) versehen sind, durch die die Gurte (59) des fahrzeugfest installierten Gurtsicherungssystems geführt sind, wobei die Gurte auf ihrem dem Benutzer abgewendeten Teil des Sitzes bzw. der Platte mit Beschlägen (60) versehen sind, mit deren Hilfe die Gurtenden am Fahrzeugsitz (55) bzw. der Platte (56) fixiert oder miteinander verbunden werden können.

15. Notfall-Stütze nach Anspruch 14, **dadurch gekennzeichnet, daß** der Fahrzeugsitz in den Fällen, in denen er als Ganzes als feste Struktur des Bergesystems dient, mittels Schnellspann-Verschlüssen am Fahrzeugboden befestigt und damit ohne Benutzung von Werkzeugen gelöst werden kann.

16. Notfall-Stütze nach Ansprüch 14 oder 15 **dadurch gekennzeichnet, daß** zur Bergung des Fahrzeugsitzes ein eine Seilrolle (62) aufweisender Hebebock (63) mit einem über die Seilrolle (62) geführten Seilzug (64) vorgesehen ist, an den der Fahrzeugsitz in seiner Gesamtheit angehängt wird.

## Claims

1. Emergency support for stabilising individual limbs or body parts for the transport or performance of operative/therapeutic measures, comprising a bolster (1, 26) made of two webs of an airtight flexible material connected at the borders thereof, and being filled with a granulate as well as provided with a valve (2) to grant an alternate evacuation and allowing an at least partial envelopment of said limbs or body parts, respectively, said bolster being provided with a trimming (8) of a clutching ribbon as a part of a clasp-and-coating joint, baffles or, respectively, throttles (7) being provided in the interior space of the bolster containing said granulate for the hindrance of the free granulates flow and hence for the maintenance of the granulates distribution effected according to the use thereof, chracterized in that a solid structure is provided having a coating trimming, that the trimming (8) is provided in the midportion of the outer surface of the bolster (1, 26) which, after applying the limbs or body parts to be stabilised, is in opposite position for the direct fixation of the bolster at the solid structure, said solid structure being formed by a salvage plate, a litter, a bucket seat of a vehicle seat or a plate being joint to the bucket seat of a vehicle seat or, respectively, being integrated therein.

2. Emergency support of claim 1, **characterized in that** the webs of the bolster are joined one with another punctiformly or in the form of a line in order to constrict the cross-section of the flow in the interior parts thereof.

3. Emergency support of claim 1 or 2, **characterized in that** the bolster is made of a thermoplasic material and the webs thereof are joined one with another punctiformly or in the form of a line by a mutual bond.

4. Emergency support of one of claims 1 to 3, **characterized in that** the bolster is subdivided in two segments by lateral incisions (3, 4) being in-line one with the other, one segment thereof serving for the embrace of the cranium and the other for the embrace of the nape/cervical region, and **in that**, in the section situated between said incisions (3, 4), said bolster is provided with at least one constriction of the cross-section (7) hindering the free granulate flow.

5. Emergency support of claim 4, **characterized in that** the bolster at the inner side of segment (6) embracing the nape/cervical region as well as at the outer side of the segement (5) embracing the cranium is provided with a clutching or coating patch (9, 10, 12) to be detachably attached to a further auxiliary appliance provided with the corresponding countertrimming, in particular to a chin support (14) and/or the filter element (17) of a respirator.

6. Emergency support of claim 5, **characterized in that**, for the fixation of the filter element (17), a fixable coating ribbon (19) equipped with a head pad (18) is provided at corresponding clutching patches (9) of the bolster (1), whereof at the surface opposite to the head pad (18) at least one coating ribbon (20, 21) is firmly sewed up at one side and whereof at the other side a clutching patch (22) for the fixation of the coating ribbon or, a the case may be, the coating ribbons (20, 21) are attached.

7. Emergency support of claim 5, **characterized in that** a chin support (14) having a countertrimming is additionally provided which can be detachably mounted at the bolster (1), the chin support (14) being provided in its section situated below the chin plate (15) with a window (16) for the performance of diagnosis or remedial measures.

8. Emergency support of one of claims 1 to 3, **characterized in that** the bolster is formed by a mat of airtight material subdivided into closed fields in the manner of a quilt, said mat comprising an envelope containing the sealing valve.

9. Emergency support of one of claims 1 to 8, **characterized in that** the bolster is provided at the lateral edges of the outer surface thereof with several clutching or coating ribbon patches of a clasp-and-coating joint.

10. Emergency support of claims 8 or 9, **characterized in that** the bolster (26) has an essentially crosslike contour and is provided with lateral flaps (29) equipped at the two sides thereof with alternate clutching and coating patches of a clasp-and-coating-joint.

11. Emergency support of one or several of claims 1 to 10, **characterized in that** the solid structure is formed of a salvage plate or litter (32) of plastics, preferably of Kevlar aramide, coated with a coating trimming (31), whereat are arranged the belts of a six-point-belt (33) having a length that allows the embrace of the bolster including the supported patient, transport loops (34) as well as a jack ring (35), the bolster (1) being fixable at said solid structure.

12. Emergency support of claims 11, **characterized in that** the essentially crosslike thoracic support (26) as well as the salvage plate are provided with covers (41, 43) that are attachable or attached one to another and are eqipped with the coating or clutching trimming, respectively, wherein the cover (41) embracing the crosslike thoracic support is provided with the lateral flaps (42) equipped on the two surfaces thereof with alternate clutching and coating patches of a clasp-and-coating-joint, whereas the cover (43) embracing the salvage plate has openings for the passage of the belts (33), the transport loops (34), the jack ring (35) and the pelvic cloth (36).

13. Emergency support of claims 12, **characterized in that** the six-point-belt (33) is provided with a central buckle (37).

14. Emergency support of claim 1, **characterized in that** the bucket seat (51, 55) of the vehicle seat or, as the case may be, the plate (52, 65) connected therewith is provided with apertures (57, 58) through which are lead the belts of the belt securing contrivance firmly installed in the vehicle, the belts being provided with braces (60) at the part of the seat or of the plate thereof that is opposite to the user, with the aid of which braces the belt ends at the vehicle seat (55) or the plate (56) can be fixed or connected one to another.

15. Emergency support of claim 14, **characterized in that** in those cases where the vehicle seat as a whole serves as a firm structure of the salvage system, the vehicle seat can be attached at the bottom of the vehicle by means of quick-acting fasteners and, hence, can be detached without the use of tools.

16. Emergency support of claim 14 or 15, **characterized in that**, for the salvage of the vehicle seat, a lifting trestle (63) is provided having a rope pulley (62) and a rope winch (64) guided over said rope pulley (62) whereat the vehicle seat is appended as a whole.

## Revendications

1. Elément de soutien de secours pour stabiliser des membres ou des parties de corps individuels pour le transport ou la réalisation de mesures opératives et/ou thérapeutiques, comportant un coussin (1, 26) en deux laizes en un matériau flexible et imperméable à l'air, rempli d'un granulat et, pour l'évacuation et le remplissage alternatifs, pourvu d'une vanne (2), ledit coussin permettant l'enveloppement au moins partiel des membres ou, selon le cas, des parties de corps, ledit coussin étant pourvu d'une paramenture (8) à partir d'un ruban cramponnant en tant que part d'un raccord crampon-tissu à longs poils, où, dans l'intérieur du coussin contenant le granulat, on a pourvu des chicanes ou, selon le cas, des étranglements (7) pour la limitation du cours libre du granulat et, par conséquant, pour la conservation de la distribution du granulat faite conformément à son emploi, **caractérisé en ce que** l'on a pourvu une structure solide fournie d'une paramenture de frise, que, pour la fixation directe du coussin à la structure solide, on a pourvu la frise (8) à la partie moyenne de la face extérieure du coussin (1, 20) qui est opposée après l'application des membres ou des parties du corps à stabiliser, où la structure solide est formée par une plaque de sauvetage, une civière, un baquet-siège d'un véhicule ou d'une plaque qui est liée avec le baquet-siège d'un véhicule ou, selon le cas, est intégrée avec celui-ci.

2. Elément de soutien de secours d'après la revendication 1, **caractérisé en ce que**, pour l'étranglement de la section transversale d'écoulement, les laizes du coussin sont liées l'une avec l'autre dans ses zones intérieures ponctuellement ou en forme de ligne.

3. Elément de soutien de secours d'après la revendication 1 ou 2, **caractérisé en ce que** le coussin consiste en matière thermoplastique et que ses laizes sont liées l'une à l'autre ponctuellement ou en forme de ligne par soudage l'une à l'autre.

4. Elément de soutien de secours d'après une des revendications 1 à 3, **caractérisé en ce que** le coussin est subdivisé en deux segments par moyen d'incisions latérales à l'alignement (3, 4), dont l'un des segments sert à l'enveloppement du crâne et l'autre à l'enveloppement de la zone nuque/cou et,dans la zone située entre lesdites incisions (3, 4), le coussin est pourvu d'au moins une des étranglements des la section transversale (7) limitant le cours libre du granulat.

5. Elément de soutien de secours d'après la revendication 4, **caractérisé en ce que**, à la face intérieure du segment enveloppant la zone nuque/cou (6) et à la face extérieure du segment enveloppant le crâne (5), le coussin est pourvu de taches cramponnantes ou à long poils (9, 10 12) d'un raccord crampon/tissu à longs poils pour la fixation détachable d'un autre dispositif auxiliaire pourvu de la correpondente contre-paramenture, en particulier d'un support de menton (14) et/ou d'une installation de filtrage (17) d'un respirateur artificiel.

6. Elément de soutien de secours d'après la revendication 5, **caractérisé en ce que**, pour la fixation de l'installation de filtrage (17), on a pourvu un ruban de frise (19) ayant un rembourrage de tête (18) et étant fixable à des taches cramponnantes (9) du coussin (1), où à la surface opposée au ruban de frise (18), au moins un ruban de frise (20, 21) est fermement arrêté à l'un des côtés et où à son autre côté, pour la fixation du ruban de frise (19), ou selon le cas des rubans de frise, on a attaché une tache cramponnante (22).

7. Elément de soutien de secours d'après la revendication 5, **caractérisé en ce que** l'on a pourvu un support de menton (14) avec un contre-paramenture qui est fixable de manière détachable au coussin (1), où ledit support de menton (14) dans sa partie située au-dessous de la plaque de menton (15) est pourvu d'une croisée (16) pour l'exécution de mesures de diagnose ou de soins.

8. Elément de soutien de secours d'après une des revendications 1 à 3, **caractérisé en ce que** le coussin est formé d'une natte en matière perméable à l'air et subdivisée en champs fermés à la manière d'une courte-pointe, ladite natte comportant un enveloppement contenent la vanne de fermeture.

9. Elément de soutien de secours d'après une des revendications 1 à 8, **caractérisé en ce que** le coussin est pourvu aux bords latéraux de sa surface extérieure de plusieures taches cramponnantes ou à long poils d'un raccord de ruban cramponnant/tissu à long poils.

10. Elément de soutien de secours d'après la revendication 8 ou 9, **caractérisé en ce que** le coussin (26) a un contour essentiellement cruciforme et est pourvu à ses deux faces de colliers de fixation (29)munis réciproquement de taches cramponnantes ou à long poils d'un raccord de ruban cramponnant/tissu à long poils.

11. Elément de soutien de secours d'après une ou plusieures des revendications 1 à 10, **caractérisé en ce que** la structure solide est formée d'une plaque de sauvetage ou d'une civière (32) en matière artificielle, de préférence en aramide Kevlar, et couvrie d'une paramenture de frise, où l'on a arrangé les ceintures d'un ceinture en six points (33) d'une longueur permettant l'enveloppement du coussin y compris le malade supporté, de dragonnes (34) et une oreille de levage(35), le coussin étant fixable à la structure solide.

12. Elément de soutien de secours d'après la revendication 11, **caractérisé en ce que** le support thoracique essentiellement cruciforme (26) aussi que la plaque de sauvetage sont pourvus avec des enveloppements (41, 43) que l'on peut joindre l'un à l'autre ou, selon le cas, sont joints l'un à l'autre et qui sont munis des paramentures de frise, ou selon le cas, des paramentures cramponnantes, l'enveloppe enveloppant le support thoracique cruciforme (41) étant muni des pattes latérales (42) qui sont fournies sur ses deux surfaces en altérance avec des plaques cramponnantes et des plaques de frise d'un raccord de crampon/tissu à longs poils, tandis que l'enveloppe (43) enveloppant la plaque de sauvetage comporte des ouvertures pour le passage des ceintures (33), des dragonnes (34), de l'oreille de levage (35) et du tissu de bassin (36).

13. Elément de soutien de secours d'après la revendication 12, **caractérisé en ce que** le ceinture en six points (33) est muni d'une serrure centrale (37).

14. Elément de soutien de secours d'après la revendication 1, **caractérisé en ce que** le baquet-siège (51, 55) du siège du véhicule, ou selon le cas, la plaque (52, 56) y attachée sont pourvus de fissures, à travers desquels on a conduit les ceintures du système de sécurité de ceinture fermement installé au véhicule, les ceintures étant fournis d'armatures (60) sur la partie du siège, ou selon le cas, de la plaque qui sont détournés de l'utilisateur, avec l'aide desquelles on peut fixer les bouts de ceinture au siège du véhicule (55), ou selon le cas, à la plaque ou l'on peut les lier l'un à l'autre.

15. Elément de soutien de secours d'après la revendication 14, **caractérisé en ce que**, aux cas où le siège du véhicule sert en tant que tout de structure solide du système de sauvetage, ledit siège de véhicule est fixé par moyen de serrures à serrage rapide au plancher du véhicule et, par conséquent, il peut être délié sans l'utilisation d'outils.

16. Elément de soutien de secours d'après la revendication 14 ou 15, **caractérisé en ce que**, pour le sauvetage du siège du véhicule, on a pourvu un vérin de levage (63) comportant une poulie (62) et une moufle à poulie (64) conduite sur ladite poulie (62), auquel est suspendu le siège dans son ensemble.
